# EUROPÄISCHE PATENTSCHRIFT

(11) **EP 1 217 968 B1**
(45) Veröffentlichungstag und Bekanntmachung des Hinweises auf die Patenterteilung: **22.03.2006**
(21) Anmeldenummer: 00931254.7
(22) Anmeldetag: 22.05.2000
(51) Int. Cl.: A61F 2/06

(54) **RADIAL EXPANDIERBARE GEFÄSSSTÜTZE**
RADIALLY EXPANDABLE VESSEL SUPPORT
TUTEUR INTRAVASCULAIRE S'ELARGISSANT RADIALEMENT

(30) Priorität: 03.08.1999 DE 19936483
(43) Veröffentlichungstag der Anmeldung: 03.07.2002
(73) Patentinhaber: Neuss, Malte, 53121 Bonn (DE); Eurocor GmbH, 53113 Bonn (DE)
(72) Erfinder: Neuss, Malte, D-53121 Bonn (DE); ORLOWSKI, Michael, 53173 Bonn (DE)
(74) Vertreter: Thiel, Christian
(86) Internationale Anmeldenummer: PCT/EP2000/004658
(87) Internationale Veröffentlichungsnummer: WO 2000/071053

(56) Entgegenhaltungen:
- EP-A- 0 876 806
- WO-A-00/06051
- WO-A-99/17680
- DE-A- 19 822 157
- DE-C- 19 740 506
- FR-A- 2 758 253
- US-A- 5 843 120

## Beschreibung

Die Erfindung betrifft eine radial aufweitbare Gefäßstütze zur Verwendung zum Offenhalten von Blutgefäßen oder sonstigen Organwegen in menschlichen oder tierischen Körpern. Diese gitterförmige Gefäßstütze besteht aus mehreren rohrförmigen Elementen mit einer zickzackförmigen Ringstruktur von geringer Breite, die mit paar weise angeordneten, gegensinnig geöffneten, Stegen miteinander verbunden sindund sternförmige Segmente bilden. In der Patentschrift EP 335 341 81 sind Gefäßstützen beschrieben, die aus langgestreckten Gliederpaaren gebildet sind. Diese Gefäßstützen werden beispielsweise in verengte oder andere Körpergefäße implantiert, um diese nach Ballondilatation dauerhaft offen zu halten. Dabei werden die Gefäßstützen in ihrem Durchmesser aufgeweitet und verkürzen sich in ihrer seitlichen Länge. Diese Verkürzung ist in der Regel unerwünscht, da dies eine Fehlpositionierung der Gefäßstütze verursachen kann. Die bekannten Gefäßstützen passen sich Bögen oder Kurven im Gefäßverlauf relativ schlecht oder gar nicht an, sodaß zusätzliche Biegeelemente vorgesehen werden müssen.

Die bekannten Gefäßstützen weisen starre röhrenförmige Abschnitte auf, die durch gelenkige Verbindungsstücke etwas biegsamer miteinander verbunden sind. Dabei können aber in diesen Bereichen, wegen der besonderen Wandbeanspruchung bei jeder Gefäßbewelgung. Hypertrophien der Gefäßwand auftreten. Andere bekannte Gefäßstützen weisen besonders bei Aufdehnung im Bereich ihres Maximaldurchmessers eine erheblich Verkürzung auf.

Gefäßstützen mit gleichsinnig gerichteten bogenförmigen Stegen zwischen den zickzackförmigen Ringelementen sind beispielsweise aus der Patentschrift DE 197 40 506 A1 bekannt. Durch die zahlreichen Stege zwischen den Ringelementen ist jene Gefäßstütze jedoch sehr steif und unflexibel, was zu einem Fehischlag bei Implantationsveisuchen bei kurvigem Gefäßverlauf führen kann.

Durch die zahlreichen bogenförmigen Stege können auch unbeabsichtigt Seitenäste des Gefäßsystems verschlossen werden, die offen bleiben sollen.

Durch die gleichsinnig gerichteten, bogenförmigen Stege auf der Zirkumferenz besteht auch der Nachteil, daß diese Gefäßstütze bei kurvigem Gefäßverlauf leicht knickt und das Gefäß teilweise oder ganz verschließt, welches mit der Gefäßstütze offengehalten werden scilte.
Die WO 00/06051 betrifft einen Stent, der sich durch ein genau definiertes Muster, welches sich hauptsächlich aus parallelen serpentinenartig geformten Elementen, die durch Brückverbindungen miteinander verbunden sind, zusammensetzt. Vorteilhaft an diesem Stent soll eine verbesserte longitudinale Flexibilität als auch eine verbesserte Dimensionsstabilität hinsichtlich einer Verkürzung des Stents entlang der Längsachse bei der Aufweitung des Stents sein.

Aufgabe der vorliegenden Erfindung ist es, eine radial aufweitbare Gefäßstütze zu schaffen, die während ihrer Aufweitung keine oder nur eine geringe Verkürzung erfährt, und bei besserer Kurvengängigkeit weniger leicht knickt und eine ausreichende Radiagfestigkeit aufweist. Auch soll die Gefäßstütze im aufgeweiteten Zustand zwischen den einzelnen Streben genügend große Querschnitte für das Offenbieiben von Seitenastabgängen des Gefäßsystems aufweisen.

Diese Aufgabe für die erwähnte Gefäßstütze wird dadurch gelöst, daß jeweils zickzackförmige Pingelemente mit mindestens einem weiteren durch paarweise angeordnete, gegensinnig geöffnete, bogenförmige Stege verbunden sind, wobei die bogenförmigen Stege an den Bögen angeordnet sind, so dass sich sternförmige Segmente ausbilden, die sich auf der Zirkumferenz paarweise gegenüberliegen.

Bei radialer Aufwertung der Gefäßstütze strecken sich die Stege entsprechend der seitlichen Verkürzung der zickzackförmigen Ringelemente in der Längsachse und vermeiden oder verringern so eine Gesamtverkürzung der Gefäßstütze. Die bogenförmigen Stege sind erfindungsgemäß bei einem oder mehreren Ringelementen jeweils paarweise in gegensätzlicher Richtung angeordnet so daß sich ein sternförmiges Aussehen

Dadurch ergibt sich trotz hoher Flexibilität durch die multizelluläre Struktur eine hohe Radialfestigkeit, die durch spiralige oder abwechselnde Anordnung von oder mehreren sternförmigen Segmenten noch erhöht wird. Um bei Gefäßstützen die Flexibilität noch weiter zu erhöhen, können beispielsweise einzelne Teilstücke mit nur zwei bogenförmigen Stegen mit gegensätzlicher Öffnungsrichtung oder mit S-förmigen Verbindungsstegen miteinander verbunden werden. Je nach Dimension, Anzahl, Anordnung und Form derartiger Verbindungsstege wird das Biegever halten der Gefäßstütze insgesamt weiter beeinflußt

Folgende Formen und Kombinationen kommen für die Verbindungsstege in Betracht: X-förmiger oder, hantelförmiger Steg, gerader oder bogenförmiger Steg, S-förmiger Steg oder ein Steg in Form einer Sinuswelle, oder ein gerader und bogenförmiger Steg.

Der Steg zwischen den einzelnen Ringelementen weist vorzugsweise einen etwas geringeren Querschnitt auf (ca. 30%), als der gerade Steg der zickzackförmigen Ringelemente.

Bei der radialen Aufdehnung der Gefäßstütze werden beispielsweise die sternförmigen Segmente auseinandergezogen und dadurch eine etwa rautenförmige Öffnung geschaffen, die vorteilshaft für den seitlichen Abgang von Gefäßen nachdilatiert werden kann und durch die eine Implantation einer weiteren Gefäßstütze in den Seitenast erfolgen kann. Zwischen zwei sternförmigen Segmenten muß mindestens ein Bogen des zickzackförmigen Ringelements frei ohne seitliche Verbindung zum nächsten Ringelement bleiben, damit sich die bogenförmigen Stege des sternförmigen Segments beim mechanischen Zusammendrücken der Gefäßstütze z.B. auf einem Ballon nicht gegenseitig behindern und damit das Einführprofil der Gefäßstütze zur Implantation durch eine Einführschleuse in den Körper möglichst klein bleibt.

Des weiteren können die zickzackförmigen Ringelemente am Rand und im Mittelbereich voneinander unterschiedliche Querschnitte aufweisen. Zur Verbesserung der Stützeigenschaften und Radialfestigkeit im Randbereich kann die Gefäßstütze an beiden Enden eine größere Stegbreite aufweisen. Zur Verbesserung der lokalen Stützeigenschaften im Bereich einer fokalen Gefäßverengung und der Radialfestigkeit kann die Gefäßstütze auch nur im Mittelbereich eine größere Stegbreite und/oder eine größeren Querschnitt und/oder mehr Verbindungstege aufweisen. Der größere Querschnitt im Mittelbereich kann z. B. durch eine weniger Material abtragende Elektropolitur erreicht werden.

Zur weiteren Erhöhung der Flexibilität können auch mehrere Teilstücke der Gefäßstütze mit zwei entgegengesetzt geöffneten bogenförmigen oder andersartig geformten Stegen jeweils paarweise miteinander verbunden werden.

Das Biegeverhalten der Gefäßstütze beim Crimpen und bei der Expansion kann eine besondere Ausbildung der Bögen der zickzack förmigen Ringelemente, beispielsweise C-förmig, haamadelförmig oder klammerförtnig, weiter verbessert werden, besonders wenn die Breite des C-förmigen oder des klammer förmigen Bogens geringer ist als die des Steges des zickzack förmigen Ringelements.

Als Material für die Gefäßstütze kann vorzugsweise eines oder mehrere biokompatible Metalle der Gruppe Niob, Platin, Stahl, Titan, einer Legierung aus Nickel-Titan, Platin-Iridium oder einer Legierung mit mindestens einem dieser Metalle, wie Platin-Iridium mit jeweils geeigneten Gewichtsprozenten, verwendet werden. Soll die Gefäßstütze selbstexpandierbar sein, wird vorzugsweise eine durch Wärmebehandlung temperaturoptimierte Nickel-Titanlegierung verwendet.

Das Metall kann zur Verbesserung des Einwachsens in die Gefäßwand mit einem biokompatiblen Material oder mit geeigneten Medikamenten zur Vermeidung von Gefäßhyperproliferation beschichtet sein oder durch Bestrahlung oder radioaktiven Zerfall eine Strahlung freisetzen.

Ferner kann die Gefäßstütze aus resorbierbaren Kunststoffen, z. B. aliphatischen Polyestern wie Polydioxanon, bestehen.

Soll die Gefäßstütze zur Schienung von Aneurysmen verwendet werden, wird sie vorzugsweise mit einem aufgenähten oder eingeflochtenen biokompatiblen Stoffgewebe aus Polyurethan, Silikon, Teflon oder Polyester versehen oder mit einer dünnwandigen Folie aus einem dieser Materialien vemäht, verschweißt, aufgeschrumpft oder verklebt.

Die rohrförmigen Körper aus Metall oder Kunststoff werden vorzugsweise aus nahtlos gezogenen Rohren gebildet, um Verspannungen und Risse zu vermeiden, wie das im Bereich von Schweißnähten der Fall wäre. Die Strukturen werden vorzugsweise durch Laserstrahl- oder Wasserstrahischneiden, Elektroerosion und Elektropolitur hergestellt.

Nachfolgend werden Ausführungsbeispiele der Erfindung anhand einer Zeichnung weiter erläutert. In der Zeichnung zeigt:
- Figur 1: in einer Darstellung des abgerollten Grundmusters eine Ausführungsform der vorliegenden Erfindung mit sternförmigen Segmenten in nicht expandiertem Zustand.
- Figur 2: einen Ausschnitt von Fig.1 mit dem für die Erfindung charakteristischen sternförmigen Segment.
- Figur 3: eine alternative Ausführungsform der vorliegenden Erfindung mit abwechselnd schräg unterhalb und oberhalb seitlich angeordneten sternförmigen Segmenten in nicht expandiertem Zustand.
- Figur 4a - d: verschiedene Ausführungsformen der zickzackförmigen Ringelemente.
- Figur 5: eine weitere alternative Ausführungsform der vorliegenden Erfindung mit spiralig, schräg untereinander angeordneten sternförmigen, Segmenten mit einer flexiblen Verbindung zweier Teilstücke mit zwei bogenförmigen Stegen.

- Figur 6a - h: verschiedenartig geformte Verbindungselemente zwischen Teilstücken. Nw Fig. 6d entspridit der Erfindung.
- Figur 7: eine weitere alternative Ausführungsform der vorliegenden Erfindung mit schräg spiralig untereinander angeordneten sternförmigen Segmenten mit einer flexiblen S-förmigen Stegverbindung zweier Teilstücke.
- Figur 8: eine weitere alternative Ausführungsform der vorliegenden Erfindung mit parallel angeordneten sternförmigen Segmenten mit hantelförmigen Stegverbindungen zwischen den aus sternförmigen Segmenten bestehenden Teilstücken .

Die in den Figuren dargestellten Gefäßstützen weisen zickzack förmige Ringelemente mit sternförmigen Segmenten und andersartig geformten Verbindungselementen auf. Daher werden aus Gründen der Übersichtlichkeit in den Figuren nur Beispiele von abgerollten Gitterstrukturen der rohrförmigen Gefäßstütze oder ein Ausschnitt davon dargestellt.

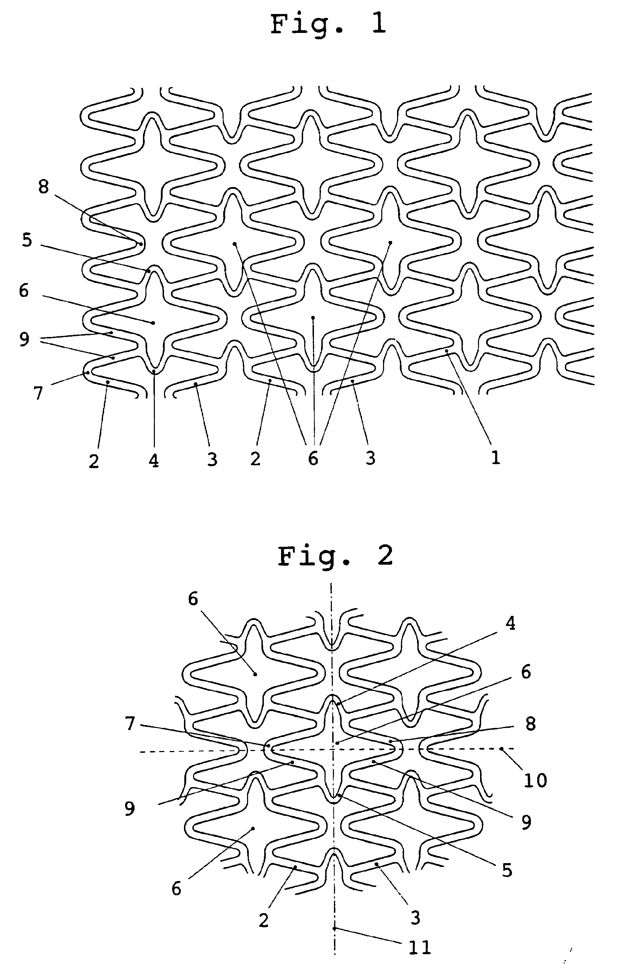

In der in Fig. 1 dargestellten Gefäßstütze 1 sind zickzackförmige Ringelemente 2 und 3 miteinander durch bogenförmige Stege 4, 5 zu sternförmigen Segmenten 6 verbunden. Die Verbindung zu dem nächsten seitlich gelegenen zickzackförmigen Ringelement 2 erfolgt ebenfalls jeweils mit einem zwischen den beiden sternförmigen Segmenten 6 seitlich angeordneten sternförmigen Segment 6 usw.

In der dargestellten Ausführungsform besteht die Gefäßstütze 1 aus zickzackförmigen Ringelementen 2, 3 mit jeweils 6 abwechselnd nach rechts 7 und nach links 8 offenen Bögen, die mit Stegen 9 zickzack förmig miteinander verbunden sind.

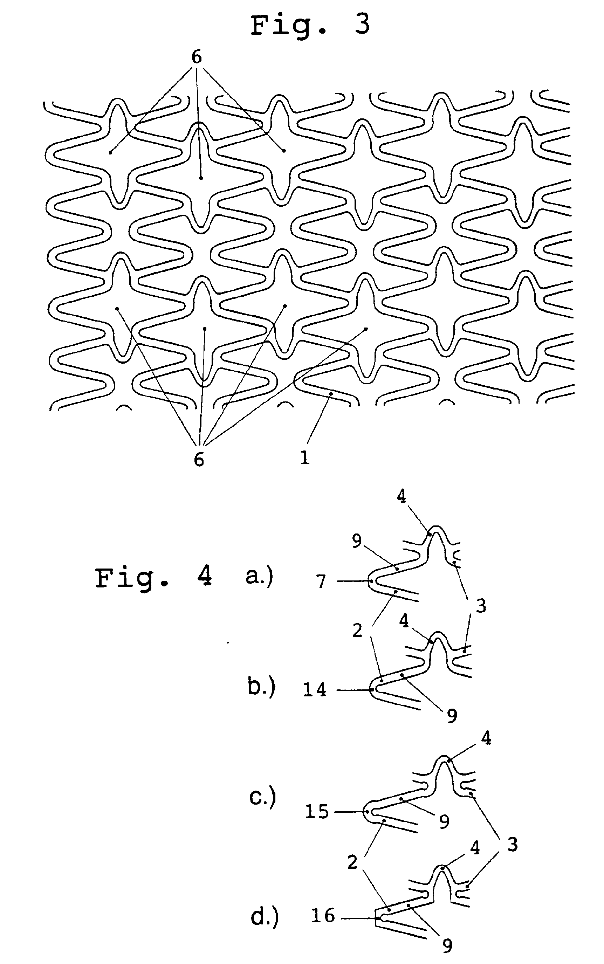

Fig. 2 stellt einen Auschnitt von Fig. 1 dar. Das zickzackförmige Ringelement 3 entspricht dem an einer gedachten Querachse 11 gespiegelten zickzackförmigen Ringelement 2. Der nach oben gewölbte bogenförmige Steg 4 entspricht dem an einer Längsachse 10, die durch den nach rechts offenen Bogen 7 des Ringelements 2 und dem nach links offenen Bogen 8 des Ringelementes 3 gebildet wird, gespiegelten u-förmigen, bogenförmigen Steg 5. Die jeweils paarweise angeordneten bogenförmigen Stege 4, 5 bilden mit dem nach rechts offenen Bogen 7 und dem nach links offenen Bogen 8 zusammen mit den Stegen 9 der Ringelemente 2, 3 jeweils zwei abwechselnd angeordnete, sternförmige Segmente 6 auf einer Längsachse 10, bzw. der Zirkumferenz der rohrförmigen Gefäßstütze.

Die sternförmigen Segmente 6 zwischen den seitlich folgenden Ringelementen 3, 2 sind jeweils seitlich zwischen den vorangegangenen sternförmigen Segmenten 6 angeordnet.

Fig. 3 zeigt eine ähnliche Ausführungsform wie Fig. 1, jedoch mit auf einer gedachten Längsachse der Gefäßstütze 1 nebeneinander seitlich abwechselnd schräg unterhalb und schräg oberhalb angeordneten sternförmigen Segmenten 6.

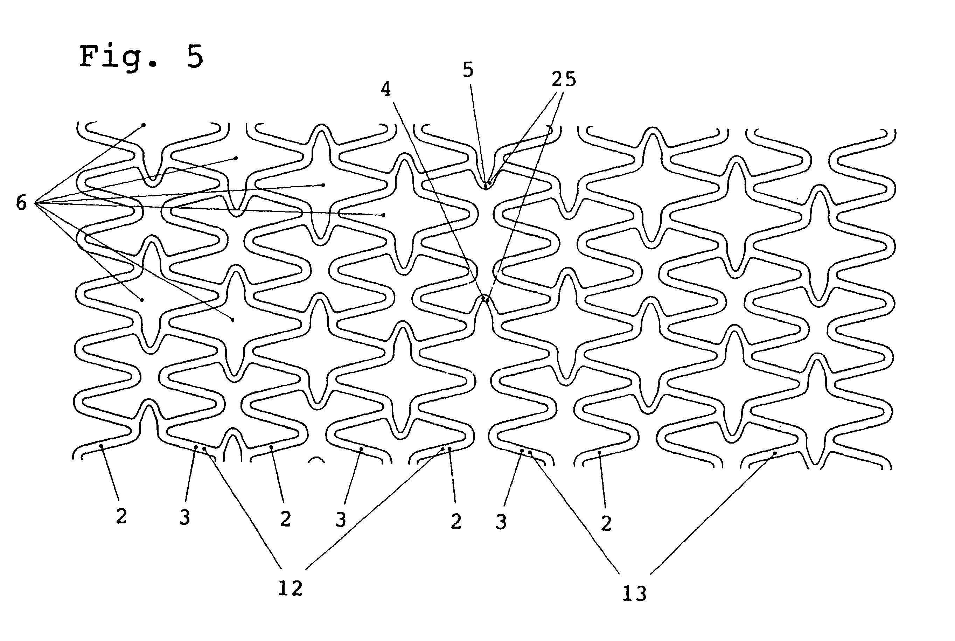

Fig. 4a zeigt eine Ausführungsform der zickzackförmigen Ringelementen 2, 3 mit nach rechts offenem Bogen 7 und einem bogenförmige Steg 4. Dabei entspricht die Breite des Bogens 7 der Breite des Steges 9.

Fig. 4b zeigt eine weitere Ausführungsform der zickzackförmigen Ringetementen 2, 3 mit nach rechts offenem C-förmigen Bogen 14 und einem bogenförmige Steg 4. Dabei ist die Breite des Breite des Bogens 14 geringer als die Breite des Steges 9.

Fig. 4c zeigt eine weitere Ausführungsform der zickzack förmigen Ringelementen 2, 3 mit nach rechts offenem haarnadelförmigen Bogen 15 und einem bogenförmige Steg 4. Dabei entspricht die Breite des Bogens 15 der Breite des Steges 9.

Fig. 4 d zeigt eine weitere Ausführungsform der zickzack förmigen Ringelementen 2, 3 mit nach rechts offenem klammerförmigen Bogen 16 und einem bogenförmige Steg 4. Dabei ist die Breite des Breite des klammerförmigen Bogens 16 geringer als die Breite des Steges 9.

Fig. 5 zeigt eine ähnliche Ausführungsform wie Fig. 3, jedoch sind auf einer gedachten Längsachse die sternförmigen Segmente 6 zwischen den zickzack förmigen Ringelementen 2, 3 seitlich schräg untereinander in Spiralform ange ordnet. Außerdem bilden mehrere durch sternförmige Segmente 6 verbundene Ringelemente 2, 3 ein Teilstück 12, das erfindungsgemäß zur

Erhöhung der Flexi-bilität mit einem weiteren Teilstück 13 nur mit zwei auf der Zirkumferenz gegenüberliegenden, bogenförmigen Stegen 4, 5 verbunden ist, die dann erfindungsgemäß ein gleichsinnig geöffnetes Biegeelement 25 bilden.

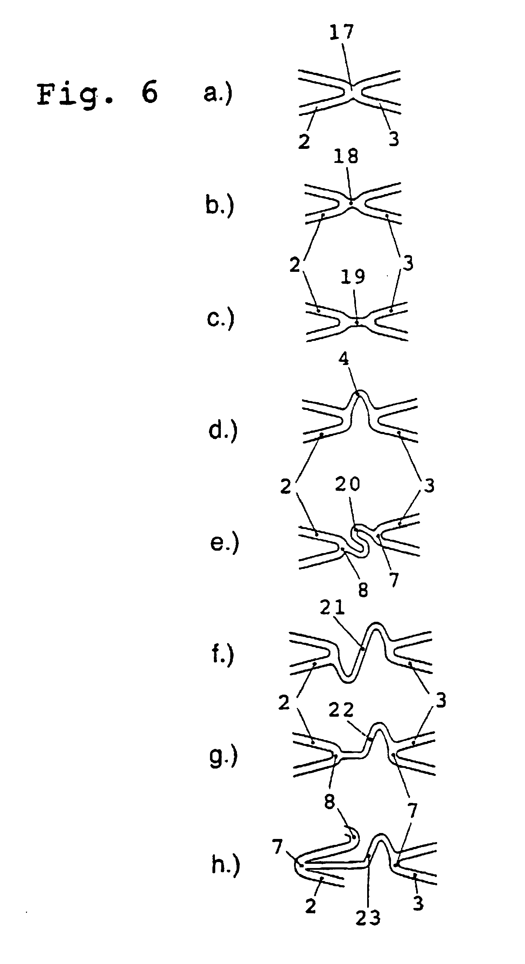

Fig. 6a zeigt einen X-förmigen Steg 17 zwischen den Zickzackförmigen Ringelementen 2, 3.

Fig. 6b zeigt einen hantelförmigen Steg 18 zwischen den zickzackförmigen Ringelementen 2, 3.

Fig. 6c zeigt einen geraden Steg 19 zwischen den zickzackförmigen Ringelementen 2, 3.

Fig. 6d zeigt einen bogenförmigen Steg 4 zwischen den zickzackförmigen Ringelementen 2.3 entsprechend der Erfindung.

Fig. 6e zeigt einen S-förmigen Steg 20 zwischen den zickzackförmigen Ringelementen 2, 3. Der S-förmige Steg 20 ist zwischen dem nach links geöffneten Bogen 8 und dem nach rechts geöffneten Bogen 7 angeordnet. Die Bögen 7, 8 liegen vorzugsweise auf unterschiedlicher Höhe.

Fig. 6f zeigt einen Steg in Form einer Sinuswelle 21 zwischen den zickzackförmigen Ringelementen 2, 3.

Fig. 6g zeigt einen geraden und bogenförmigen Steg 22 als Kombination der Fig. 6c und 6d zwischen den zickzack förmigen Ringelementen 2. 3. Der Steg 22 ist zwischen dem nach links geöffneten Bogen 8 und dem nach rechts geöffneten Bogen 7 angeordnet.

Fig. 6h zeigt einen geraden und bogenförmigen Steg 23 ähnlich wie bei Fig. 6g mit längerem geraden Anteil, jedoch ist der Steg 23 links innen zwischen dem nach rechts geöffneten Bogen 7 des zickzackförmigen Ringelements 2 und rechts außen an dem nach rechts geöffneten Bogen 7 des zickzackförmigen Ringelementes 3 angeordnet.

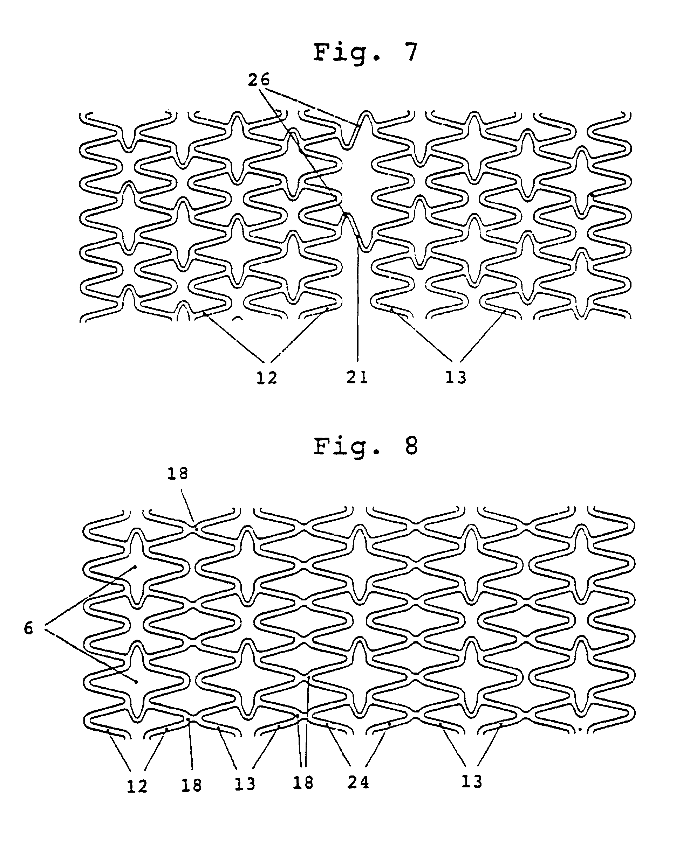

Fig. 7 zeigt eine ähnliche Ausführungsform wie Fig. 5, jedoch werden die beiden Teilstücke 12, 13 mit einem Biegeelement 26 aus Stegen in Form einer Sinuswelle 21 miteinander verbunden.

In Fig. 8 ist eine weitere alternative Ausführungsform der vorliegenden Erfindung dargestellt mit parallel angeordneten sternförmigen Segmenten 6. Dabei ist jeweils ein Teilstück 12, bestehend aus sternförmigen Segmenten 6 mit dem seitlich folgenden Teilstück 13 mit mehreren hantelförmigen Stegen 18 verbunden. Zwischen dem Mittelbereich 24 und den angrenzenden Teüstücken 13 kann auch jeweils ein hantelförmiger Steg 18 angeordnet sein.

Aus der vorstehenden Beschreibung und der Darstellung von Ausführungsbeispielen wird deutlich, daß sich die Erfindung nicht auf die in den Ansprüchen oder der Beschreibung genannten Merkmalskombinationen beschränkt, sondern im Rahmen der Erfindung auch andere Kombinationen der aufgeführten Merkmale denkbar sind.

### Bezugszeichenliste

- 1.: Gefäßstütze
- 2.: zickzackförmiges Ringelement
- 3.: zickzackförmiges Ringelement
- 4.: bogenförmiger Steg
- 5.: bogenförmiger Steg
- 6.: sternförmiges Segment
- 7.: nach rechts offener Bogen
- 8.: nach links offener Bogen
- 9.: Steg
- 10.: Längsachse
- 11.: Querachse
- 12.: Teilstück
- 13.: Teilstück
- 14.: C-förmiger Bogen
- 15.: haamadelförmiger Bogen
- 16.: klammerförmiger Bogen
- 17.: X-förmiger Steg
- 18.: hantelförmiger Steg
- 19.: gerader Steg
- 20.: S-förmiger Steg
- 21.: Steg in Form einer Sinuswelle
- 22.: gerade und bogenförmiger Steg
- 23.: gerade und bogenförmiger Steg
- 24.: Mittelbereich
- 25.: Biegeelement
- 26.: Biegeelement

## Patentansprüche

1. Radial aufweitbare Gefäßstütze, welche eine Vielzahl von miteinander flexibel verbundenen zickzackförmigen Ringelementen (2, 3) bestehend aus mit Stegen (9) verbundenen Bögen (7,8) aufweist, welche eine Gefäßstütze (1) mit einem proximalen und einem distalen Ende und einer Längsachse definieren, wobei die zickzackförmigen Ringelemente (2, 3) quer zur Längsachse der Gefäßstütze nebeneinander angeordnet sind,
**dadurch gekennzeichnet, dass** jedes zickzackförmige Ringelement (2, 3) mit mindestens einem weiteren zickzackförmigen Ringelement (2, 3) durch paarweise angeordnete, gegensinnig geöffnete, bogenförmige Stege (4, 5) verbunden ist, wobei die bogenförmigen Stege (4,5) an den Bögen (7,8) angeordnet sind, so dass sich sternförmige Segmente (6) ausbilden, die sich auf der Zirkumferenz paarweise gegenüberliegen (Fig.1).

2. Radial aufweitbare Gefäßstütze nach Anspruch 1, **dadurch gekennzeichnet, daß** die Breite der bogenförmigen Stege (4, 5) 10 bis 50% kleiner ist als die Breite der Stege (9) der zickzackförmigen Ringelemente (2, 3).

3. Radial aufweitbare Gefäßstütze nach einem der Ansprüche 1 bis 2, **dadurch gekennzeichnet, daß** die Breite der bogenförmigen Stege (4, 5) 30% kleiner ist als die Breite der Stege (9) der zickzackförmigen Ringelemente (2, 3).

4. Radial aufweitbare Gefäßstütze nach einem der Ansprüche 1 bis 3, **dadurch gekennzeichnet, daß** die zickzackförmigen Ringelemente (2, 3) an den Enden jeweils zu Bögen (7, 8) abgerundet sind.

5. Radial aufweitbare Gefäßstütze nach einem der Ansprüche 1 bis 4, **dadurch gekennzeichnet, daß** die Breite der zickzackförmigen Ringelement (2, 3) im Bereich der Bögen (7, 8) größer ist als im Bereich der Stege (9).

6. Radial aufweitbare Gefäßstütze nach Anspruch 1, **dadurch gekennzeichnet, daß** die Breite der Stege (9) der zickzackförmigen Ringelemente (2, 3) und/oder der bogenförmigen Stege (4, 5) an den seitlichen Enden der Gefäßstütze größer ist als im Mittelbereich.

7. Radial aufweitbare Gefäßstütze nach einem der Ansprüche 1 bis 5, **dadurch gekennzeichnet, daß** die Breite und/oder der Querschnitt der Stege (9) und/oder der Bögen (7, 8) der zickzackförmigen Ringelemente (2, 3) und/oder der bogenförmigen Stege (4. 5) und damit die Radialkraft im Mittelbereich der Gefäßstütze größer ist als an den Enden.

8. Radial aufweitbare Gefäßstütze nach Anspruch 1, **dadurch gekennzeichnet, daß** die sternförmigen Segmente (6) zwischen den seitlich folgenden zickzackförmigen Ringelementen (3, 2) jeweils seitlich gegeneinander versetzt (Fig. 1) angeordnet sind.

9. Radial aufweitbare Gefäßstütze nach Anspruch 1, **dadurch gekennzeichnet, daß** die sternförmigen Segmente (6) zwischen den zickzackförmigen Ringelementen (2, 3) jeweils seitlich versetzt und aneinander angrenzend (Fig. 3) übereinander angeordnet sind.

10. Radial aufweitbare Gefäßstütze nach Anspruch 1, **dadurch gekennzeichnet, daß** die sternförmigen Segmente (6) zwischen den zickzackförmigen Ringelementen (2, 3) seitlich schräg untereinander in Spiralform (Fig. 5) angeordnet sind.

11. Radial aufweitbare Gefäßstütze nach Anspruch 8 oder 9, **dadurch gekennzeichnet, daß** mehrere durch sternförmige Segmente (6) verbundene Ringelemente (2, 3) ein Teilstück (12) bilden, das mit einem oder mehreren Teilstücken (13) nur mit jeweils zwei auf der Zirkumferenz gegenüberliegenden, bogenförmigen Stegen (25) verbunden ist.

12. Radial aufweitbare Gefäßstütze nach Anspruch 8 oder 9, **dadurch gekennzeichnet, daß** mehrere durch sternförmige Segmente (6) verbundene Ringelemente (2, 3) ein Teilstück (12) bilden, das mit einem oder mehreren weiteren Teilstücken (13) nur mit jeweils zwei oder mehreren auf der Zirkumferenz gegenüberliegenden S-förmigen Stegen (26) verbunden ist.

13. Radial aufweitbare Gefäßstütze nach einem der Ansprüche 1 bis 12, **dadurch gekennzeichnet, daß** sie im wesentlichen aus einem oder mehreren aus Stahl, Tantal, Titan, Niob, Platin oder einer Legierung aus mindestens einem dieser Metalle mit mindestens einem weiteren Metall gebildet ist.

14. Radial aufweitbare Gefäßstütze nach einem der Ansprüche 1 bis 13, **dadurch gekennzeichnet, daß** sie aus einer Nickel-Titan-Legierung besteht, die durch Wärmebehandlung selbstexpandierbar gemacht ist.

15. Radial aufweitbare Gefäßstütze nach einem der Ansprüche 1 bis 12, **dadurch gekennzeichnet, daß** sie aus einem resorbierbaren Stoff, vorzugsweise Kunststoff, besteht.

16. Radial aufweitbare Gefäßstütze nach einem der Ansprüche 1 bis 15, **dadurch gekennzeichnet, daß** sie mit einem biokompatiblen Material beschichtet ist.

17. Radial aufweitbare Gefäßstütze nach einem der Ansprüche 1 bis 16, **dadurch gekennzeichnet, daß** sie mit geeigneten Medikamenten dauerhaft zur Vermeidung von Intimahyperproliferation der Gefäßwand beschichtet ist.

18. Radial aufweitbare Gefäßstütze nach Anspruch 17, **dadurch gekennzeichnet, daß** die Beschichtung die geeigneten Medikamente zur Vermeidung von Intimahyperproliferation der Gefäßwand langsam freisetzt.

19. Radial aufweitbare Gefäßstütze nach einem der Ansprüche 1 bis 15, **dadurch gekennzeichnet, daß** sie eine Beschichtung aufweist, die durch radioaktiven Zerfall Strahlung zur Vermeidung oder Reduktion von Intimahyperproliferation der Gefäßwand freisetzt.

20. Radial aufweitbare Gefäßstütze nach einem der Ansprüche 1 bis 19, **dadurch gekennzeichnet, daß** sie mit einem biokompatiblen Stoffgewebe aus Polyurethan, Silikon, Teflon oder Polyester oder einer dünnwandigen Folie aus einem dieser Materialien versehen ist.

## Claims

1. A radially expandable vessel support having a multitude of zigzag shaped annular elements (2, 3) mutually connected in a flexible way and consisting of bows (7, 8) connected by bars (9), said annular elements (2, 3), defining a vessel support (1) with a proximal and a distal end and a longitudinal axis, where said zigzag shaped annular elements (2, 3) are arranged side by side across said longitudinal axis, **characterized in that** each zigzag shaped annular element (2, 3) is linked, respectively, to at least one more zigzag shaped annular element (2, 3) by means of bars (4, 5) arranged in a pairwise manner, which are bow shaped and curved in opposing directions, said bow shaped bars (4, 5) being arranged at the bows (7, 8) such that star shaped segments (6) are formed, which are located in a pairwise manner on opposite sides of the circumference (fig.1).

2. A radially expandable vessel support according to claim 1, **characterized in that** the width of the bow shaped bars (4, 5) is between 10% and 50% smaller than the width of the bars (9) of the zigzag shaped annular elements (2, 3).

3. A radially expandable vessel support according to any of claims 1 to 2, c. i. that the width of the bow shaped bars (4, 5) is 30 % smaller than the width of the bars (9) of the zigzag shaped annular elements (2, 3).

4. A radially expandable vessel support according to any of claims 1 to 3, **characterized in that** the zigzag shaped annular elements (2, 3) are made round at the ends to form bows (7, 8).

5. A radially expandable vessel support according to any of claims 1 to 4, **characterized in that** the width of the zigzag shaped annular elements (2, 3) is larger in the range of the bows (7, 8) than in the range of the bars (9).

6. A radially expandable vessel support according to claim 1, **characterized in that** the width of the bars (9) of the zigzag shaped annular elements (2, 3) and/or of the bow shaped bars (4, 5) is larger at the lateral ends than in the central region of the vessel support.

7. A radially expandable vessel support according to any of claims 1 to 5, **characterized in that** the width and/or the cross section of the bars (9) and/or of the bows (7, 8) of the zigzag shaped annular elements (2, 3) and/or of the bow shaped bars (4, 5), and therewith also the radial force, is larger in the central region of the vessel support than at its ends.

8. A radially expandable vessel support according to claim 1, **characterized in that** the star shaped segments (6) between adjacent zigzag shaped annular elements (3, 2) are arranged, respectively, in a sidewise displaced fashion (fig. 1).

9. A radially expandable vessel support according to claim 1, **characterized in that** the star shaped segments (6) between the zigzag shaped annular elements (2, 3) are arranged, respectively, in a fashion where they are displaced sidewise and stacked contiguously above one another (fig. 3).

10. A radially expandable vessel support according to claim 1, **characterized in that** the star shaped segments (6) between the zigzag shaped annular elements (2, 3) are arranged sidewise among one another at an oblique angle in the shape of a spiral (fig. 5).

11. A radially expandable vessel support according to claim 8 or 9, **characterized in that** several annular elements (2, 3) linked by star shaped segments (6) form a sector (12) which is linked to one or several sectors (13) only by means of two each of bow shaped bars (25) which are arranged in opposing positions on the circumference.

12. A radially expandable vessel support according to claim 8 or 9, **characterized in that** several annular elements (2, 3) linked by star shaped segments (6) form a sector (12) which is linked to one or several further sectors (13) only by means of two or several S-shaped bars (26) which are arranged in opposing positions on the circumference.

13. A radially expandable vessel support according to any of claims 1 to 12, **characterized in that** it is essentially made of one or several of steel, tantalum, titanium, niobium, platinum, or of an alloy consisting of at least one of these metals and at least one further metal.

14. A radially expandable vessel support according to any of claims 1 to 13, **characterized in that** it is made of a nickel-titanium alloy which has been made self expanding by a heat treatment.

15. A radially expandable vessel support according to any of claims 1 to 12, **characterized in that** it is made from a resorbable material, preferably a plastic.

16. A radially expandable vessel support according to any of claims 1 to 15, **characterized in that** it is coated with a biocompatible material.

17. A radially expandable vessel support according to any of claims 1 to 16, **characterized in that** it is permanently coated with suitable medicaments to avoid intimal hyperproliferation of the vascular wall.

18. A radially expandable vessel support according to claim 17, **characterized in that** the coating will slowly release the medicaments suitable to avoid intimal hyperproliferation of the vascular wall.

19. A radially expandable vessel support according to any of claims 1 to 15, **characterized in that** is has a coating which, by way of radioactive decay, releases radiation to avoid or reduce intimal hyperproliferation of the vascular wall.

20. A radially expandable vessel support according to any of claims 1 to 19, **characterized in that** it is provided with a biocompatible fabric made of polyurethane, silicone, Teflon, or polyester, or with a thin walled foil made of one of these materials.

## Revendications

1. Tuteur intravasculaire s'élargissant radialement lequel présente un grand nombre d'éléments annulaires en forme de zigzag liés les uns aux autres de façon flexible se composant de courbures (**7,8**) reliées à l'aide de parties médianes courbées (**9**), lesquels définissent un tuteur intravasculaire (**1**) avec une extrémité proximale et une extrémité distale et un axe longitudinal, les éléments annulaires en forme de zigzag (**2, 3**) étant agencés l'un à côté de l'autre de façon transversale à l'axe longitudinal du tuteur intravasculaire, **caractérisé en ce que** chaque élément annulaire en forme de zigzag (**2, 3**) est relié à l'aide d'au moins un autre élément annulaire (**2, 3**) en forme de zigzag par des parties médianes courbées (**4, 5**)ouvertes en sens inverse, agencées par paire, les parties médianes courbées (**4, 5**) étant agencées, au niveau de la courbure (**7**, **8**), de telle sorte que des segments en forme d'étoile (**6**) se forment, lesquels se font face par paire sur la circonférence (figure 1).

2. Tuteur intravasculaire s'élargissant radialement selon la revendication 1, **caractérisé en ce que** la largeur des parties médianes courbées (**4, 5**) est inférieure de 10 à 50 % à la largeur des parties médianes courbées (**9**) des éléments annulaires en forme de zigzag (**2, 3**).

3. Tuteur intravasculaire s'élargissant radialement selon l'une quelconque des revendications 1 à 2, **caractérisé en ce que** la largeur des parties médianes courbées (**4, 5**) est inférieure de 30 % à la largeur des parties médianes courbées (**9**) des éléments annulaires en forme de zigzag (**2**, **3**).

4. Tuteur intravasculaire s'élargissant radialement selon l'une quelconque des revendications 1 à 3, **caractérisé en ce que** les éléments annulaires en forme de zigzag (**2**, **3**) sont arrondis au niveau de chacune des courbures (**7**, **8**).

5. Tuteur intravasculaire s'élargissant radialement selon l'une quelconque des revendications 1 à 4, **caractérisé en ce que** la largeur de l'élément annulaire (**2, 3**) en forme de zigzag est plus grand au niveau des courbures (**7, 8**) qu'au niveau des parties médianes (**9**).

6. Tuteur intravasculaire s'élargissant radialement selon la revendication 1, **caractérisé en ce que** la largeur des parties médianes (**9**) des éléments annulaires en forme de zigzag (**2, 3**) et/ou des parties médianes courbées (**4, 5**) est plus importante au niveau des extrémités latérales du tuteur intravasculaire que dans la partie intermédiaire.

7. Tuteur intravasculaire s'élargissant radialement selon l'une quelconque des revendications 1 à 5, **caractérisé en ce que** la largeur et/ou la section des parties médianes (**9**) et/ou des courbures (**7, 8**) des éléments annulaires en forme de zigzag (**2, 3**) et/ou des parties médianes courbées (**4, 5**) et, par conséquent, la force radiale est plus importante dans la partie intermédiaire du tuteur intravasculaire qu'au niveau des extrémités.

8. Tuteur intravasculaire s'élargissant radialement selon la revendication 1, **caractérisé en ce que** les segments en forme d'étoile (6) sont, à chaque fois, agencés en quinconce les uns par rapport aux autres de façon latérale (figure 1) entre les éléments annulaires (3, 2) en forme de zigzag se suivant latéralement.

9. Tuteur intravasculaire s'élargissant radialement selon la revendication 1, **caractérisé en ce que** les segments en forme d'étoile (6) sont, à chaque fois, agencés les uns sur les autres en quinconce et de façon adjacente (figure 3) les uns aux autres entre les éléments annulaires en forme de zigzag (2, 3).

10. Tuteur intravasculaire s'élargissant radialement selon la revendication 1, **caractérisé en ce que** les segments en forme d'étoile (6) sont agencés entre les éléments annulaires en forme de zigzag (2, 3) latéralement, de façon oblique les uns sous les autres selon une forme hélicoïdale (figure 5).

11. Tuteur intravasculaire s'élargissant radialement selon la revendication 8 ou 9, **caractérisé en ce que** plusieurs éléments annulaires (2, 3) reliés par des segments en forme d'étoile (6) forment un bloc (12), qui est relié avec un ou plusieurs blocs (13) n'ayant, à chaque fois, que deux parties médianes courbées (25) se faisant face sur la circonférence.

12. Tuteur intravasculaire s'élargissant radialement selon la revendication 8 ou 9, **caractérisé en ce que** plusieurs éléments annulaires (**2, 3**) reliés par des segments en forme d'étoile (**6**) forment un bloc (**12**), qui est relié avec un ou plusieurs autres blocs (**13**) n'ayant, à chaque fois, que deux parties médianes courbées (**26**) en forme de **S** se faisant face sur la circonférence ou plus.

13. Tuteur intravasculaire s'élargissant radialement selon l'une quelconque des revendications 1 à 12, **caractérisé en ce qu'**il est formé principalement à partir d'un ou de plusieurs métaux choisis parmi l'acier, le tantale, le titane, le niobium, le platine ou d'un alliage d'au moins l'un de ces métaux avec au moins un autre métal.

14. Tuteur intravasculaire s'élargissant radialement selon l'une quelconque des revendications 1 à 13, **caractérisé en ce qu'**il est réalisé à partir d'un alliage nickel-titane, qui est fabriqué par un traitement thermique pouvant s'auto-développer.

15. Tuteur intravasculaire s'élargissant radialement selon l'une quelconque des revendications 1 à 12, **caractérisé en ce qu'**il se compose d'une matière résorbable, de préférence une matière synthétique.

16. Tuteur intravasculaire s'élargissant radialement selon l'une quelconque des revendications 1 à 15, **caractérisé en ce qu'**il est enduit d'un matériau biocompatible.

17. Tuteur intravasculaire s'élargissant radialement selon l'une quelconque des revendications 1 à 16, **caractérisé en ce qu'**il est enduit, de façon durable, d'un médicament approprié permettant d'éviter l'hyperprolifération de la tunique interne des parois vasculaires.

18. Tuteur intravasculaire s'élargissant radialement selon la revendication 17, **caractérisé en ce que** le revêtement libère lentement les médicaments appropriés permettant d'éviter l'hyperprolifération de la tunique interne des parois vasculaires.

19. Tuteur intravasculaire s'élargissant radialement selon l'une quelconque des revendications 1 à 15, **caractérisé en ce qu'**il présente un revêtement, qui libère par sa désagrégation radioactive une radiation permettant d'éviter ou de réduire l'hyperprolifération de la tunique interne de la paroi vasculaire.

20. Tuteur intravasculaire s'élargissant radialement selon l'une quelconque des revendications 1 à 19, **caractérisé en ce qu'**il est pourvu d'un tissu imprégné de matériau biocompatible choisi parmi le polyuréthane, la silicone, le téflon ou le polyester ou d'une pellicule à fine paroi réalisée à partir d'un de ces matériaux.
